# EUROPEAN PATENT APPLICATION

(11) **EP 0 597 151 A1**
(43) Date of publication of application: **18.05.1994**
(21) Application number: 92203444.2
(22) Date of filing: 10.11.1992
(51) Int. Cl.: C07C 53/02, C07C 51/02, C07C 51/15, C07C 51/41, C07C 51/48

(54) **Method for preparing formic acid**

(71) Applicant: UNIVERSITEIT TWENTE, NL-7500 AE Enschede (NL)
(72) Inventor: Engel, Dirk Coenraad, NL-7511 GH Enschede (NL); Van Swaaij, Willibrordus Petrus Maria, NL-7581 BZ Losser (NL); Versteeg, Geert Frederik, NL-7752 NC Hengelo (NL)
(74) Representative: van der Saag, Johannes

(57) **Abstract**

The invention is concerned with a method for preparing formic acid wherein an aqueous solution of an aminoformate is subjected to distillation, thus decomposing the aminoformate and recovering the resulting formic acid, wherein an aminoformate is used which decomposes into formic acid and an amino compound which is either an amine with a volatility higher than the volatility of the formic acid in the aqueous solution containing aminoformate and formic acid, or ammonia.

This method can be suitably applied in a continuous process for the production of formic acid from carbon dioxide and hydrogen. Three of these continuous processes are described.

## Description

The present invention is concerned with a method for preparing formic acid wherein an aqueous solution of an aminoformate is subjected to distillation, thus decomposing the aminoformate and recovering the resulting formic acid.

Such a method is known from European patent 0,248,259 and a publication by Braca, G., Ricci, A., and Sbrana, G. (la chimica E l'industria V. 71. N. November 11, 1989). In these publications the formic acid is recovered by stripping it with steam from a solution of a hydroxyalkylammoniumformate. The hydroxyalkylammoniumformate is decomposed into formic acid and hydroxyalkylamine. The formic acid is stripped into the vapour phase, while the hydroxyalkylamine remains in the liquid phase. This process has several disadvantages. The hydroxyalkylammoniumformate is a stable salt at temperatures below 150°C, and it easily dehydrates to formamide. Furthermore, unless excessive quantities of steam are used, normally only part of the formic acid can be recovered.

Now, a method allowing an effective and complete recovery of the formic acid from the aqueous aminoformate solution, while avoiding an uneconomical waste of steam, is provided. This method is characterized by the use of an aminoformate which decomposes into formic acid and an amino compound which is either an amine with a volatility higher than the volatility of the formic acid in the aqueous solution containing aminoformate and formic acid, or ammonia. The distillation may be a steam distillation, but is preferably a normal distillation, in which no steam is used. The formic acid is recovered from the aqueous solution by means of an extractive agent, preferably simultaneously with the distillation.

The catalysts used in EP 248,259 may be used in the method according to the invention. However, the use of an extra catalyst is not necessary, because formic acid itself catalyses the reaction. The use of only formic acid as a catalyst is preferred, because it avoids the introduction of foreign compounds into the system.

The amino compound must fulfill several conditions to function in the method according to the invention.
1) It is soluble in water.
2) An aqueous solution of its formate salt is decomposable at a temperature lower than or equal to the boiling point of the aqueous aminoformate solution.
3) It has a higher volatility than the formic acid in the aqueous solution of aminoformate and formic acid, and is stable under process conditions.

In order to have a volatility higher than the volatility of the formic acid in the aqueous solution containing formic acid and the aminoformate, the total number of carbon atoms in the amino compound is preferably not higher than 6.

The amino compound is of the formula R₁R₂R₃N, wherein the substituents R₁, R₂, and R₃, which may be the same or different, are H, or optionally substituted hydrocarbyl groups, while any two or all R₁, R₂ and R₃ may form part of a ring. Suitably the hydrocarbyl group is an aliphatic or cycloaliphatic group. Substituted hydrocarbyl groups may contain for example nitrogen, oxygen, bromine or fluorine.

Hydroxyalkylamines are excluded from the method according to the invention because their boiling point is too high. Compounds like CH₃-NH-O-CH₃ may be suitable. Aldehydes and ketones can be used in theory, but in general they appear to be either unstable under process conditions or to have a too high boiling point. The melting point of these substances is in most cases already above 100°C. Halogen-substituted alkylamines like NH₂-CH₂-CH₂F and CH₃-NH-Br are suitable. Diamines appear to have either a too high boiling point or decompose in water. Compounds like methyl hydrazine, 1,1-dimethyl hydrazine, and 1,2-dimethyl-hydrazine, although they are strictly speaking no amines, can also be used. Mixtures of said amino compounds may also be used.

R₁, R₂ and R₃ are preferably all either H, or unsubstituted lower alkyl groups. Examples of suitable trialkylamines are monomethyldiethylamine, dimethylmonoethylamine, trimethylamine and triethylamine. Mixtures of said amino compounds may also be used. Preferred amines are mono-, di- and trimethyl- and -ethylamines. Ammonia, trimethyl- and triethylamine are most preferred.

The requirements for the choice of a proper extractive agent for the formic acid are:
1) It must have a higher boiling point than the aqueous solution containing aminoformate and formic acid, i.e. above 100°C, however, in general above 150°C and preferably above 200°C (at atmospheric pressure).
2) It has to be sufficiently non-polar to assure immiscibility with said aqueous solution containing the aminoformate and formic acid under the conditions maintained in the extractive stripper.
3) It must be polar enough to be capable of withdrawing formic acid from the aqueous phase and simultaneously non-polar enough to prevent uptake of the amino compound and formate ions, hence it has to be a non-solvent for the aminoformate.

The pKa of the extractive agent is also of importance, because it influences the selectivity of the extractive agent for formic acid. The pKa is preferably between 4 and 9. Further, it is important that the extractive agent does not form or dissolve ionic compounds. In this respect, the dielectric constant, the most direct measure of the polarity of the extractive agent, should be regarded. The dielectric constant of the extractive agent is preferably lower than 10-15. Other properties of a suitable extractive agent are a high capacity for formic acid, a low capacity for water, a high thermal stability, a low heat capacity and viscosity, a low melting point and a significant difference between the densities of the extractive agent and water.

Extractive agents which satisfy the aforesaid conditions comprise (mixtures of) most of the organic solvents frequently encountered in the extractive or azeotropic separation of formic acid/water mixtures. Examples of suitable organic solvents are dialkylamides and tri-alkyl amines, see for instance DE-OS 2,545,658 (1977), EP Appl. 17,866 (1980) and DE-OS 3,428,319 (1986).

The trialkyl amines with a high boiling point are preferably used, because they have a high selectivity for formic acid as compared to water, and more important, to the aminoformate. The minimum total number of carbon atoms is 9 (derived from the boiling point of tripropylamine (156°C)). Of these tri-n-octylamine is preferably used. Of the dialkylamides, di-n-butylformamide is preferred.

The method is carried out at a temperature between 60 and 200°C, preferably between 80 and 150°C. In this range both the decomposition reaction of the aminoformate and the extraction of formic acid are favoured. The pressure in the reaction vessel is between 1 and 20 bar (abs), preferably between 1 and 10 bar (abs). However, the exact pressure used depends on the temperature, and the concentration and type of aminoformate used.

The method can be carried out batch-wise or continuously. A continuous operation is preferred. The initial concentration of the aminoformate solution depends, inter alia, on the method of preparation of the aminoformate solution. In general, the aminoformate concentration will be between 5 and 85% by weight (i.e. between 1 and 60 mol %) and preferably between 10 and 60% by weight (i.e. between 5 and 25 mol %). The aminoformate solutions used in the method of the invention can for example be obtained by the carbonylation of primary and secondary amines known in the state of the art (page 2, lines 46 and 47 of EP 0,248,259). However, we have found that they can be advantageously obtained with the production method described below, which results in a continuous production of formic acid from CO₂ and H₂.

This method can be summarized in the following reactions:
1) reaction of an aminocompound with carbon dioxide and water to an aminobicarbonate.

   R₁R₂R₃N + CO₂ + H₂O R₁R₂R₃NH HCO₃,

   in which R₁, R₂ and R₃ have the above-mentioned meaning;
2) reaction of an alkali metal- or ammoniumhydrogen carbonate with hydrogen to a formate salt and water

   MHCO₃ + H₂ MOOCH + H₂O,

   in which M is an alkali metal or ammonium;
3) reaction of the aminobicarbonate from step 1 and the formate salt from step 2 to form an aminoformate and a hydrogen carbonate which can be recycled to step 2.

   R₁R₂R₃ NH HCO₃ + MOOCH R₁R₂R₃HNOOCH + MHCO₃;
4) decomposition of the aminoformate into formic acid and the amino compound with the method according to the invention.

   R₁R₂R₃ NHOOCH R₁R₂R₃N + HCOOH.

This sequence of steps can be carried out according to several different methods, such as:
A: a method using a low boiling tertiary amine, comprising the following steps:
   a) in a first stage a low boiling tertiary amine, carbon dioxide and water are reacted together to produce an aqueous amine bicarbonate solution;
   b) in a second stage hydrogen is reacted in the presence of a heterogeneous catalyst with an aqueous solution of a bicarbonate salt of an alkali metal or ammonium to form the corresponding formate salt and water;
   c) optionally the heterogeneous catalyst is separated in a third stage from said aqueous solution of step b by some conventional means of liquid/solid separation and thereafter recycled to step b;
   d) in a fourth stage the amine bicarbonate obtained in step a is converted into the corresponding formate by means of ion exchange across a membrane with the alkali metal formate or ammonium formate formed in step b, which results in the recovery of the corresponding bicarbonate;
   e) in a fifth stage the carbon dioxide and low boiling amine that are released by the decomposition of the unconverted amine bicarbonate residual of step d, are recycled to step a;
   f) in a sixth stage the amine formate is converted into formic acid and the amine by the method according to the invention.
B: a method using ammonia, comprising the following steps:
   a) in a first stage ammonia, carbon dioxide and water are reacted together to produce an aqueous ammonium bicarbonate solution;
   b) in a second stage hydrogen is reacted in the presence of a heterogeneous catalyst with the aqueous ammonium bicarbonate solution to form ammonium formate and water;
   c) optionally the heterogeneous catalyst is separated in a third stage from the aqueous solution of step b by some conventional means of liquid/solid separation and thereafter recycled to step b;
   d) in a fourth stage the carbon dioxide and low boiling amine that are released by the decomposition of the unconverted ammonium bicarbonate residual of step c are recycled to step a;
   e) in a fifth stage the ammonium formate is converted into formic acid and ammonia by the method according to the invention.
C: a method using a low boiling amine and an ammonium-bicarbonate comprising the following steps:
   a) in a first stage a low boiling amine, carbon dioxide and water are reacted together to produce an aqueous amine bicarbonate solution;
   b) in a second stage hydrogen is reacted in the presence of a heterogeneous catalyst with an aqueous solution of ammonium bicarbonate to form the corresponding formate salt and water;
   c) optionally the heterogeneous catalyst is separated in a third stage from the aqueous solution of step b by some conventional means of liquid/solid separation and thereafter recycled to step b;
   d) in a fourth stage the amine bicarbonate obtained in step a is converted into the corresponding formate by means of a base exchange with the ammonium formate formed in step b, which results in the recovery of the corresponding bicarbonate;
   e) in a fifth stage the carbon dioxide and low boiling amine that are released by the decomposition of the unconverted amine bicarbonate residual of step d, are recycled to step a;
   f) in a sixth stage the aminoformate is converted into formic acid and the amine by the method according to the invention.

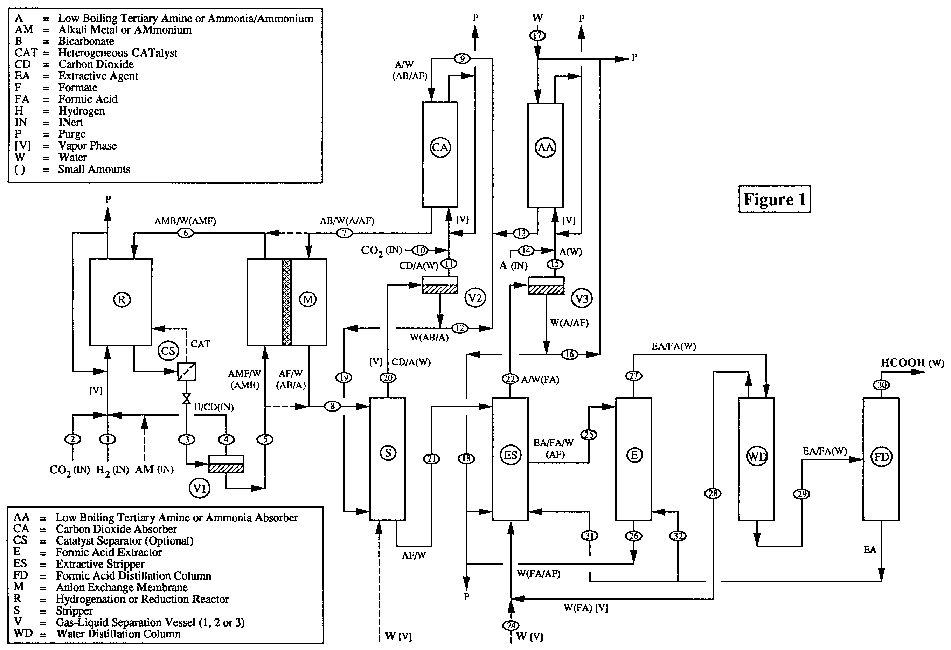

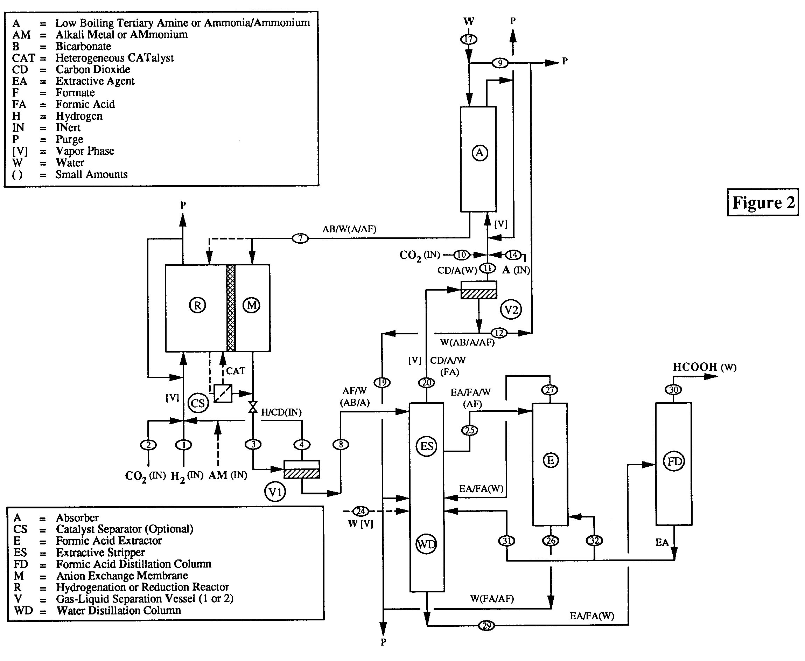

These methods can all be carried out following the same production schemes, illustrated in figures 1 and 2.

According to figure 1, a recycle stream containing a diluted aqueous solution of a low boiling amino compound and some traces of the unrecovered aminoformate coming from separation vessel V3, is fed via line 16 to the amino absorber AA, in which a vapor phase of amino compound and some water is contacted with said recycle stream via line 15, at a preferred temperature between 20 and 60°C. This contact may be in cocurrent flow, but is desirably carried out countercurrently. Depending on the solubility of the amino compound in the aqueous solution the absorber may be operated under increased pressure. However the absorption is preferably conducted at atmospheric pressure. The concentrated aqueous solution of the amino compound thus obtained is combined via line 13 with recycle line 12, which consists essentially of a mixture of aminobicarbonate and the amino compound in water. The resulting solution is fed via line 9 into carbon dioxide absorber CA, in which a gas mixture consisting of carbon dioxide, some amino compound and water is led to form a concentrated or, more preferably, saturated or supersaturated aqueous solution of principally aminobicarbonate. The gaseous mixture originates from recycle line 11 and freshly admixed carbon dioxide via line 10. Any inert components present can be kept below a desired concentration level by controlling the amount of purged gas. The preferred operating conditions correspond with those described for the aminoabsorber. The applied type of equipment for the absorbers AA and CA may range from a simple bubble column or stirred vessel to a column specifically designed for efficient gas-liquid contact, which is provided with, for example, sieve-trays, or structural or random packings. The obtained aminobicarbonate solution is transferred via line 7 to anion exchange membrane M, in order to recover the formate produced in hydrogenation reactor R and to provide simultaneously new bicarbonate via line 6, which can be recycled together with the aqueous alkali metal or ammonium salt solution to said hydrogenation reactor, as represented by line 5.

The dashed lines near the feed and product streams of the membrane unit indicate the special case wherein the alkali metal is replaced by ammonium and the low boiling amino compound is ammonia. In that case the membrane unit will be, of course, superfluous. The hydrogenation may be carried out at a temperature between 0 and 150°C, although a temperature between 20 and 80°C is preferred. Fresh hydrogen is fed via line 1. Furthermore, a stream of carbon dioxide is added to the gaseous feed via line 2 in order to make up the losses caused by the gas purge that is provided after the reactor. The hydrogen, which may be used either as a pure gas or as a mixture with other gases such as carbon dioxide, nitrogen, methane or ammonia, is widely available on an industrial scale from a considerable number of sources, e.g., from
1) coke oven gas.
2) a plant specifically designed for the production of hydrogen such as a hydrocarbon gasification or catalytic reforming unit.
3) a plant in which hydrogen is made as a byproduct, such as in steam cracking of hydrocarbons or carbon-monoxide, in acetylene production, in thermal and catalytic cracking or in dehydrogenation and oxidation processes.
4) other chemical processes, such as by the conversion of metals, ammonia, methanol or hydrogen sulfide.
5) units which apply methods of water decomposition such as electrolysis.

The carbon dioxide may either be carbon dioxide itself, which is widely available on an industrial scale, or carbon dioxide obtained from carbonate, carbamate or bicarbonate sources, or from mixtures thereof. Carbonates, carbamates or bi-carbonates can be used directly. Carbon dioxide may be used as a liquid or as a solid, though preferably as a gas. In case of using a gaseous stream of carbon dioxide, this can be, e.g., from
1) another plant such as a fermentation process.
2) a carbon dioxide containing off-gas from another part of the process.
3) a unit specifically designed for the manufacture of carbon dioxide such as the hot potassium carbonate process.
4) a production facility which makes carbon dioxide as a byproduct, such as an ammonia plant.
5) natural sources such as a gas field.
6) a furnace fuel gas.
7) oxidation or steam reforming of a carbon monoxide containing gas stream.

It is preferred to use carbon dioxide and hydrogen at partial pressures which are as high as practicable and economic. The use of high partial pressures of hydrogen is desirable because the reaction rate and yield of the formate salt increase with increasing partial pressure. The partial pressure of carbon dioxide and, in the event of replacing the alkali metal by ammonium, ammonia, is less critical, but suitably their partial pressure may be up to 20 bar. On the other hand, the hydrogen partial pressure may suitably be up to 100 bar. Conveniently the partial pressure of ammonia and/or carbon dioxide is from 0.1 to 10 bar and that of hydrogen from 1 to 50 bar. The hydrogenation reactor may be any of the gas-liquid-solid reactors which are generally known in the art, hence either a slurry reactor (stirred vessel, bubble column or three phase fluidized bed), a trickle flow reactor or a packed bubble column may be selected. In the event of using a reactor whereby the catalyst is kept in suspension, as in the case of slurry reactors, a conventional catalyst separation CS is necessary whereas this step is trivial in case of using an immobilized catalyst, viz. when a trickle flow reactor or packed bubble column is chosen. The pressure in the product stream coming from the reactor or catalyst separator via line 3, is released via a throttle whereby the dissolved gas components escape for the larger part from the liquid phase in separation vessel V1. Depending on the actual reactor pressure, this may also be done stepwise. After repressurization, the resulting gas mixture can be returned to the reactor via line 4. Subsequently, the product stream coming from the membrane unit via line 8 is fed into the top of stripper S. The solution contains essentially aminoformate and unconverted aminobicarbonate in water. In the stripper the unconverted aminobicarbonate is recovered from said aqueous solution according to the reversible reaction (1) by operating at a preferred temperature between 20 and 100°C at either atmospheric or subatmospheric pressure. The applied type of apparatus for the stripper may range from a simple flash vessel comprising one theoretical equilibrium stage, to a multistage column whereby an optional feature is provided to add steam into the bottom of the column for enhanced mass transfer and favoured state of equilibrium. The vapor phase escaping from the top of the stripper via line 20, consists of a mixture of the liberated carbon dioxide, the amino compound and water, which is directly cooled to its dew point, whereafter the condensed water can partly be recycled from separation vessel V2 to the bottom of the stripper via line 19. If the process conditions are properly chosen, a virtually pure aqueous aminoformate solution can be withdrawn from the bottom of the stripper, which is subsequently passed to the top of the extractive stripper ES via line 21. This column is operated at a temperature that causes the aminoformate to decompose significantly into formic acid and the low boiling amino compound, which is desirably between 80 and 150°C. Steam may be fed into the bottom of the column via the dashed line 24 to strip the low boiling amino compound selectively from the aqueous liquid phase into the vapor phase. Depending on the temperature maintained in the extractive stripper, this steam may be supplied at atmospheric or increased pressure and may either be saturated or superheated. However, preferably there should be no net addition of water to the aminoformate solution, because otherwise a continous dilution of the solution will occur. The amount of steam added through line 24 should be adjusted in this way. However, the use of steam is in most cases not preferred.

In the top of the extractive stripper a vapor phase develops, containing for the larger part the amino compound and water, and is removed via line 22. Most of the water and some of the concomitantly evaporated formic acid can be recovered by condensation. Part of the condensed liquid may suitably be recycled to the bottom of the extractive stripper via line 18. The residual amount will be passed to the amino absorber via line 16 and a small amount may be purged as an effluent. The amino compound and water lost in this way, are made up via line 14 and 17 respectively. Via line 31 a suitable extractive agent is added in the bottom of the extractive stripper to withdraw the formic acid selectively from the aqueous liquid phase by counter-current flow in relation to the denser aqueous liquid phase. Due to the relatively high temperature, the miscibility of the two phases may be considerable and, as a result, a substantial amount of water dissolves in the second liquid phase which principally consists of extractive agent and recovered formic acid. The extractive liquid phase loaded with formic acid, water and traces of aminoformate, is withdrawn from the top of the column via line 25 but preferably as a side-stream, and transferred to the top of the extractor E. Depending on the specific process conditions and the desired purity of the formic acid, the use of an extractor will not always be necessary. It is preferred to add pure extractive agent to the extractive stripper on several places along the height of the stripper, because the concentration of formic acid in solution diminishes along the height of the stripper, while the concentration of formic acid in the extractive agent increases. The addition of extra extractive agent has a positive influence on the displacement of the equilibrium. The extractor is operated at a considerably lower temperature than the extractive stripper in order to improve the selectivity in favour of formic acid. In this way most of the water along with the remaining traces of aminoformate can be removed from the bottom of the extractor via line 26. The optimum temperature in the extractor generally depends on the type of extractive agent used, but is preferably chosen between 20 and 80°C.

The resulting extract is passed via line 27 to water distillation column WD, wherein the rest of the water is removed overhead via line 28. Hereafter practically pure formic acid can be obtained via line 30 by distillation of the resulting mixture of extractive agent and formic acid, as represented by line 29. If still further purification of the product is desired, this can be achieved by simple distillation afterwards, which is not shown here. The recovered extractive agent can suitably be recycled to the extractive stripper via line 31 and to the extractor via line 32. The section for the reprocessing of the formic acid / extractive agent / water mixture is described in more detail elsewhere, for which reference is made to EP Appl. 17,866 (1980). Alternatively, if the catalyst is suspended in the liquid phase, it may also be possible to combine the stages c) and d) by utilizing at the same time the ion exchange membrane as a catalyst filter. In that event the ion exchange in the membrane unit has to be carried out at conditions close to those employed in the hydrogenation reactor. Moreover, a combination of stage b), c) and d) may be possible, in which the ion exchange membrane is incorporated in the hydrogenation reactor, see figure 2. If the occurring conversion to formate after the hydrogenation and ion exchange is relatively high, it might as well be advantageous to combine the decomposition of aminobicarbonate and aminoformate in a single column, as is shown in figure 2 by extractive stripper ES. As a consequence, the adsorption of carbon dioxide and the amino compound might also be accomplished in a single apparatus, as represented by absorber A.

### Example 1 - steam distillation

In a steam distillation unit comprising a steam generator, a flask having a volume of 250 ml, a condenser and an oil bath with temperature control, saturated steam was led through 150 ml of a 2 mol/l NH₄OOCH solution for 1 hour at 100°C. Two distillation fractions were obtained. Titrimetric analysis with 0.1 mol/l HCl solution resulted in 0.17 and 0.10 mol/l NH₃, respectively. Determining the composition of the residu by means of titration with 0.5 mol/l NaOH solution yielded 0.05 mol/l HCOOH and 1.35 mol/l NH₄OOCH.

### Example 2 - distillation

In the apparatus of example 1 the distillation was repeated with 175 ml of a 4.5 mol/l NH₄OOCH solution, however, in this case without supplying steam. 4 distillation fractions were obtained, the results of which with respect to the titrimetric analysis (carried out as in example 1) are given in the following table. The residu (50 ml) contained 1.93 mol/l HCOOH and 11.46 mol/l NH₄OOCH.

| Fraction (no.) | Volume (ml) | Temp. (°C) | NH₃ (mol/l) | NH₄OOCH (mol/l) |
|---|---|---|---|---|
| 1 | 25 | 100 | 0.67 | - |
| 2 | 50 | 102 | 0.35 | - |
| 3 | 40 | 104 | 0.52 | - |
| 4 | 20 | 106 | 1.31 | 0.16 |

### Example 3 - extraction

A mixture of 7.97 g (0.126 mol) NH₄OOCH and 5.67 g (0.123 mol) HCOOH was dissolved in 50.12 g (2.78 mol) H₂O in a three-necked flask with a volume of 250 ml. The flask was equipped with a reflux cooler, thermometer, heating mantle and stirrer. Before adding 81.3 g (0.230 mol) of extractive agent (tri-n-octylamine) the composition of the aqueous mixture was determined titrimetrically with the aid of a 0.5 mol/l NaOH solution. The mixture contained 1.93 mol/l HCOOH and 2.05 mol/l NH₄OOCH. The two-phase system was subsequently heated to 100°C and intensively stirred at this temperature for 1 hour. Titrimetric analysis of a sample of the aqueous phase showed that it contained 0.97 mol/l HCOOH and 2.22 mol/l NH₄OOCH.

### Example 4 - hydrogenation

20.5 g (0.259 mol) NH₄HCO₃, 502.3 g (27.9 mol) H₂O and 7.1 g of a catalyst containing 5% Palladium by weight on active carbon were added to a glass reactor having a volume of 1000 ml. The reactor was heated after evacuation to 60°C and pressurized up to 10 bar (abs)H₂. The conditions were maintained for 12 hours while stirring the reaction mixture intensively. Afterwards a catalyst-free liquid sample was taken and by titrimetric analysis with 0.5 mol/l HCl solution, NH₄OOCH could be determined in a concentration of 0.32 mol/l. This corresponds with a conversion of 65% on a total carbon base.

### Example 5 - distillation and simultaneous extraction using a low boiling amine

In the apparatus of example 2 approximately 85 ml of an aqueous mixture containing 4.54 mol/l (CH₃)₃HNOOCH-(trimethylamine formate) and 2.80 mol/l HCOOH was subjected to distillation and simultaneous extraction with 199.4 g (0.564 mol)tri-n-octylamine for 3 quarters of an hour. In this case, however, the flask had a volume of 500 ml and about 2 extra gas/liquid equilibrium stages had been created before the distillate was successively condensed and collected. 3 distillation fractions were obtained, see the table presented below. The composition of the aqueous mixtures was again determined titrimetrically, this time with both a 5 mol/l NaOH and a 5 mol/l HCl solution. All 3 distillation fractions consisted of trimethylamine and water only. At the end of the experiment it was observed that there was no residu present as a separate aqueous liquid phase.

| Fraction (No.) | Temperature (°C) | N(CH₃)₃ (mol/l) |
|---|---|---|
| 1 | 100-101 | 2.65 |
| 2 | 102-103 | 3.12 |
| 3 | <95 | 2.32 |

### Example 6 - distillation and simultaneous extraction using ammonia

The experiment described in example 5 was repeated with about 150 ml of an aqueous solution of 3.95 mol/l NH₄OOCH. This time the distillation was carried out with 158.2 g(1.006 mol) of the extractive agent di-n-butylformamide. After boiling the mixture for about 5 quarters of an hour, 4 distillation fractions were collected, which have been analyzed in the usual way. Only the last fraction contained, in addition to ammonia, some ammonium formate, as can be seen from the table presented below. The residual aqueous solution (approximately 25 ml) crystallized partially upon cooling to ambient temperature.

| Fraction (no.) | Temperature (°C) | NH₃ (mol/l) | NH₄OOCH (mol/l) |
|---|---|---|---|
| 1 | 98-99 | 0.84 | - |
| 2 | 99-100 | 0.68 | - |
| 3 | 100-101 | 1.01 | - |
| 4 | >101 | 3.00 | 0.12 |

## Claims

1. A method for preparing formic acid wherein an aqueous solution of an aminoformate is subjected to distillation, thus decomposing the aminoformate and recovering the resulting formic acid, **characterized in that** an aminoformate is used which decomposes into formic acid and an amino compound which is either an amine with a volatility higher than the volatility of the formic acid in the aqueous solution containing aminoformate and formic acid, or ammonia.

2. A method according to claim 1, **characterized in that** the formic acid is recovered during the distillation by simultaneous extraction with a suitable extractive agent.

3. A method according to claim 1 or 2, **characterized in that** the amino compound is of the formula R₁R₂R₃N, wherein the substituents R₁, R₂, and R₃, which may be the same or different, are H, or optionally substituted hydrocarbyl groups, while any two or all R₁, R₂ and R₃ may form part of a ring, while the maximum total number of carbon atoms in the amino compound is 6.

4. A method according to claim 3, **characterized in that** the amino compound is either trimethyl- or triethylamine, or ammonia.

5. A method according to any one of the preceding claims 2-4, **characterized in that** the extractive agent used is a trialkylamine in which the minimum total number of carbon atoms is 9.

6. A method according to any one of the preceding claims 2-4, characterized in that the extractive agent used is di-n-butylformamide.

7. A method according to any one of the preceding claims, **characterized in that** it is carried out in a continuous process at a temperature between 80 and 150°C, under a pressure of 1 to 10 bar (abs).

8. A method for the production of formic acid from carbon dioxide and hydrogen in a continuous process, using a low boiling tertiary amine, wherein
a) in a first stage a low boiling tertiary amine, carbon dioxide and water are reacted together to produce an aqueous amine bicarbonate solution;
b) in a second stage hydrogen is reacted in the presence of a heterogeneous catalyst with an aqueous solution of a bicarbonate salt of an alkali metal or ammonium to form the corresponding formate salt and water;
c) optionally the heterogeneous catalyst is separated in a third stage from the aqueous solution of step b by some conventional means of liquid/solid separation and thereafter recycled to step b;
d) in a fourth stage the amine bicarbonate obtained in step a is converted into the corresponding formate by means of ion exchange across a membrane with the alkali metal formate or ammonium formate formed in step b, which results in the recovery of the corresponding bicarbonate;
e) in a fifth stage the carbon dioxide and low boiling amine that are released by the decomposition of the unconverted amine bicarbonate residual of step d are recycled to step a;
f) in a sixth stage the amine formate is converted into formic acid and the amine by the method according to any of the claims 1-7.

9. A method for the production of formic acid from carbon dioxide and hydrogen in a continuous process, using ammonia, wherein
a) in a first stage ammonia, carbon dioxide and water are reacted together to produce an aqueous ammonium bicarbonate solution;
b) in a second stage hydrogen is reacted in the presence of a heterogeneous catalyst with the aqueous ammonium bicarbonate solution to form ammonium formate and water;
c) optionally the heterogeneous catalyst is separated in a third stage from the aqueous solution of step b by some conventional means of liquid/solid separation and thereafter recycled to step b;
d) in a fourth stage the carbon dioxide and ammonia that are released by the decomposition of the unconverted ammonium bicarbonate residual of step c are recycled to step a;
e) in a fifth stage the ammonium formate is converted into formic acid and ammonia by the method according to any of the claims 1-7.

10. A method for the production of formic acid from carbon dioxide and hydrogen in a continuous process, using a low boiling amine, wherein
a) in a first stage a low boiling amine, carbon dioxide and water are reacted together to produce an aqueous amine bicarbonate solution;
b) in a second stage hydrogen is reacted in the presence of a heterogeneous catalyst with an aqueous solution of ammonium bicarbonate to form the corresponding formate salt and water;
c) optionally the heterogeneous catalyst is separated in a third stage from the aqueous solution of step b by some conventional means of liquid/solid separation and thereafter recycled to step b;
d) in a fourth stage the amine bicarbonate obtained in step a is converted into the corresponding formate by means of a base exchange with the ammonium formate formed in step b, which results in the recovery of the ammonium bicarbonate;
e) in a fifth the stage carbon dioxide and low boiling amine that are released by the decomposition of the unconverted amine bicarbonate residual of step d, are recycled to step a;
f) in a sixth stage the amine formate is converted into formic acid and the amine by the method according to any of the claims 1-7.
